# EUROPÄISCHE PATENTSCHRIFT

(11) **EP 3 258 983 B1**
(45) Veröffentlichungstag und Bekanntmachung des Hinweises auf die Patenterteilung: **20.03.2019**
(21) Anmeldenummer: 16703551.8
(22) Anmeldetag: 09.02.2016
(51) Int. Cl.: A61M 1/06

(54) **BRUSTPUMPE UND KAPPE HIERFÜR**
BREAST PUMP AND CAP FOR SAME
TIRE-LAIT ET COUVERCLE CORRESPONDANT

(30) Priorität: 20.02.2015 EP 15155895
(43) Veröffentlichungstag der Anmeldung: 27.12.2017
(73) Patentinhaber: Medela Holding AG, 6340 Baar (CH)
(72) Erfinder: RIGERT, Mario, 6033 Buchrain (CH); FISCHER, René, 8057 Zürich (CH)
(74) Vertreter: Clerc, Natalia
(86) Internationale Anmeldenummer: PCT/EP2016/052723
(87) Internationale Veröffentlichungsnummer: WO 2016/131680

(56) Entgegenhaltungen:
- WO-A2-01/47577
- US-A- 5 542 921
- US-A1- 2007 060 873
- US-A1- 2007 135 761
- US-A1- 2010 324 478
- US-A1- 2013 294 942
- US-A1- 2014 094 748

## Beschreibung

### TECHNISCHES GEBIET

Die vorliegende Erfindung betrifft eine Brustpumpe zum Abpumpen menschlicher Muttermilch und eine Kappe hierfür.

### STAND DER TECHNIK

Brustpumpen zum Abpumpen von menschlicher Muttermilch sind hinlänglich bekannt. Sie weisen eine manuell oder mittels eines Elektromotors betriebene Vakuumpumpe zur Erzeugung eines Unterdrucks, eine mit der Vakuumpumpe verbundene Brusthaube zur Auflage auf die Mutterbrust sowie einen mit der Brusthaube verbundenen Milchsammelbehälter zum Sammeln der abgepumpten Milch auf.

US 5 776 098 beschreibt eine Brustpumpe mit einer Pumpmembran, welche über eine Antriebsstange mit einem Elektromotor verbunden ist. Die Pumpmembran ist in einer steifen Platte auf einer Seite einer weichen Tasche befestigt und von einer steifen Kappe überdeckt. Die Kappe liegt mit einem inneren Flansch dichtend an einem umlaufenden Rand der Pumpmembran an. Mit einem äusseren Flansch ist sie auf der steifen Platte aufgeschnappt.

WO 2001/47577 offenbart eine Brustpumpe zum gleichzeitigen Abpumpen beider Brüste. Sie weist hierfür zwei flexible Pumpmembranen auf, welche in einer Vertiefung in der Oberseite des Gehäuses angeordnet sind. Diese Vertiefung lässt sich mit einem Deckel verschliessen. Die Pumpmembranen sind mit je einer Antriebsachse eines motorisch angetriebenen Mechanismus verbunden und werden nach Massgabe einer vorgegebenen Bewegungskurve nach unten und oben bewegt. Auf diese Pumpmembranen lässt sich je eine steife Kappe stülpen, welche über eine Saugleitung mit der Brusthaube verbunden ist. Zwischen Kappe und sich bewegender Pumpmembran ist eine Pumpkammer gebildet, in welcher ein Unterdruck erzeugt wird. Entsprechend der Bewegungskurve entsteht so eine Saugkurve, welche einen sich zyklisch ändernden Unterdruck an die Mutterbrust anlegt. In einer Ausführungsform sind die Kappen fest mit dem Deckel verbunden und werden beim Schliessen des Deckels auf die zugehörige Pumpmembran gedrückt. In einer anderen Ausführungsform ist eine Schutzmembran zwischen Kappe und Pumpmembran angeordnet, welche eine dichte Verbindung ermöglicht und zudem eine Kontamination der Pumpmembran mit abgepumpter Milch oder Bakterien verhindert. Die Schutzmembran weist auf ihrer Innenseite eine umlaufende Schulter und eine umlaufende Wulst auf, welche einen Kanal bilden. Der Kanal ist auf einem vorstehenden Rand der Pumpmembran aufgenommen, wodurch gleichzeitig eine luftdichte Passung und die Befestigung der Kappe auf der Pumpmembran erfolgt. Ein Rückschlagventil ist in der Pumpmembran angeordnet, um beim ersten Hub allfällige Luft zwischen Pumpmembran und Schutzmembran entweichen zu lassen. Diese Pumpe ist auf dem Markt unter dem Namen "Symphony" bekannt und hat sich sehr bewährt. Sie eignet sich insbesondere für die Verwendung in Spitälern und in der Vermietung, da sie dank der Schutzmembran von mehreren Müttern verwendet werden kann. Dichtungen zwischen zwei weichen Teilen sind jedoch nicht so einfach, so dass die Herstellung der Kappe, der Schutzmembran und der Pumpmembran engen Massvorgaben unterliegt und entsprechend fundierte Kenntnisse erfordert.

Weitere relevante Brustpumpen aus dem Stand der Technik sind in den Dokumenten US2014/0094748 A1 und US 2007/0060873 A1 offenbart.

Mittlerweile sind im Stand der Technik diverse Brusthaubeneinheiten bekannt, welche eine Medientrennmembran im Bereich der Brusthaube aufweisen und welche dadurch die Pumpe auf diese Art und Weise vor Verunreinigungen schützen. Eine derartige Brusthaubeneinheit zeigen beispielsweise US 2004/0087898 und WO 2008/057218.

### DARSTELLUNG DER ERFINDUNG

Es ist deshalb eine Aufgabe der Erfindung, eine Brustpumpe mit einer alternativen luftdichten Verbindung zwischen steifer Kappe und flexibler Pumpmembran zu schaffen. Diese Aufgabe lösen eine Brustpumpe mit den Merkmalen des Patentanspruchs 1 sowie eine Kappe einer derartigen Brustpumpe mit den Merkmalen des Patentanspruchs 7.

Die erfindungsgemässe Brustpumpe zum Abpumpen menschlicher Muttermilch weist eine motorisch angetriebene flexible Pumpmembran und eine auf diese stülpbare steife Kappe auf. Zwischen Pumpmembran und Kappe ist eine Pumpkammer zur Erzeugung eines Unterdrucks gebildet. Die Kappe ist über eine Saugleitung mit einer Brusthaubeneinheit verbindbar oder sie ist bereits fest mit ihr verbunden, wodurch der in der Pumpkammer erzeugte Unterdruck in die Brusthaubeneinheit übertragbar ist. Die Pumpmembran weist einen umlaufenden Kragen auf mit einer Stufe. Der Kragen weist ferner einen seitlich vorstehenden Vorsprung auf und die Kappe besitzt auf ihrer Innenseite einen umlaufenden Anlagebereich. Die Kappe weist auf ihrer Innenseite mindestens ein Formschlusselement auf, welches beabstandet zum umlaufenden Anlagebereich angeordnet ist und welches mit dem Vorsprung der Pumpmembran einen Formschluss bildet, so dass die Kappe eingeschnappt auf der Pumpmembran aufliegt. Der Anlagebereich der Kappe und die Pumpmembran im Bereich der Stufe stehen in einem gemeinsamen Reibschluss und bildet eine Dichtung, so dass das eingeschnappte Aufliegen der Kappe auf der Pumpmembran ausreicht, um die Kappe dichtend auf der Pumpmembran und der Pumpe zu halten und die Pumpkammer zu bilden.

Somit ist die Brustpumpe durch einfaches Auflegen und Einschnappen der steifen Kappe auf der flexiblen Pumpmembran funktionsfähig zusammengesetzt. Die Kappe lässt sich somit gleich einfach oder sogar noch einfacher Aufsetzen als die bisher mit der Symphony-Pumpe verwendeten Kappen mit Schutzmembran. Es sind keine weiteren Befestigungsmittel notwendig, um die Kappe mit dem Gehäuse zu verbinden. Insbesondere muss die Kappe nicht mit Fixierungsmitteln wie Schrauben und Einrasthaken am Gehäuse der Pumpe fixiert werden. Die Kappe ist lediglich mit der Pumpmembran verbunden.

In der erfindungsgemässen Brustpumpe muss nicht mehr zwingend eine Schutzmembran zwischen Kappe und Pumpmembran vorhanden sein. Vorzugsweise befinden sich bei der erfindungsgemässen Brustpumpe sogar keine Schutzmembran und auch kein anderes Teil zwischen Kappe und Pumpmembran, so dass sich Kappe und Pumpmembran mindestens im Bereich des Formschlusses und des Reibschlusses unmittelbar und direkt kontaktieren.

Vorzugsweise ist eine definierte Fügekraft notwendig, um die Kappe auf der Pumpmembran einschnappen zu lassen. Diese Fügekraft beträgt vorzugsweise 5 bis 50 N. Vorzugsweise ist ein Anschlag vorhanden, bis zu welchem die Kappe auf die Pumpmembran gestülpt werden kann. Dies erleichtert der Mutter zu erkennen, ob die Kappe korrekt und vollständig aufgesetzt ist.

Das Einschnappen der Kappe auf die Pumpmembran erfolgt vorzugsweise nicht lautlos sondern mit einem Einschnappgeräusch, welches wiederum oder alternativ zum Anschlag eine Rückmeldung an die Mutter gibt. Die Mutter erhält dadurch die Bestätigung, dass die Brustpumpe einsatzbereit ist.

In einer bevorzugten Ausführungsform ist zwischen dem Anlagebereich und dem mindestens einen Formschlusselement der Kappe ein umlaufender Kanal gebildet, welcher den Kragen der Pumpmembran umgibt und welcher den Vorsprung aufnimmt.

Das mindestens eine Formschlusselement ist ein Schnappelement, welches unter den Vorsprung der Pumpmembran eingreift.

Diese Kappe lässt sich somit auch auf die Pumpmembran der bekannten Symphony-Pumpe dichtend und funktionsfähig aufsetzen, ohne dass eine zusätzliche Schutzmembran verwendet werden muss.

Um bei einem hohen Überdruck ein Abheben der Brusthaube zu vermeiden, insbesondere beim ersten Hub, ist in der Brustpumpe ein Entlüftungsventil angeordnet, welches die Pumpkammer mit einer Aussenseite verbindet.

In bevorzugten Ausführungsformen ist das Entlüftungsventil in der Kappe angeordnet. Zusätzlich oder alternativ ist das Entlüftungsventil in einem die Saugleitung mit der Kappe verbindenden Stecker oder in der Brusthaubeneinheit oder in der Saugleitung angeordnet.

Die erfindungsgemässe Kappe zur Verwendung in einer Brustpumpe ist über eine Saugleitung mit einer Brusthaubeneinheit verbindbar oder mit ihr verbunden. Sie weist auf ihrer Innenseite einen umlaufenden Anlagebereich und auf ihrer Innenseite mindestens ein Formschlusselement auf, welches beabstandet zum umlaufenden Anlagebereich angeordnet ist zur Bildung eines Formschlusses mit einem Vorsprung einer Pumpmembran der Brustpumpe. Die Kappe ist im Bereich des Anlagebereichs zur Bildung eines Reibschlusses mit der Pumpmembran ausgebildet, so dass ein Aufliegen der Kappe auf der Pumpmembran ausreicht, um die Kappe dichtend auf der Pumpmembran zu halten.

Diese Kappe lässt sich in der bekannten Symphony-Pumpe oder in einer anderen Brustpumpe mit geeignet ausgebildeter Pumpmembran verwenden. Insbesondere kann sie in Gebrauchslage der Brustpumpe in vertikaler Richtung der Brustpumpe oben auf der Pumpmembran aufliegen. Ist die Pumpmembran in einer vertikal verlaufenden Seitenfläche angeordnet, so kann sie auch in dieser Lage seitlich auf die Pumpenmembran aufgesteckt sein. Andere Raumpositionen in Bezug auf die Pumpmembran sind ebenfalls je nach Ausgestaltung der Brustpumpe möglich.

Die Kappe weist vorzugsweise einen domähnlichen Grundkörper auf. Das heisst, er ist halbkugelförmig und weist eine abgeflachte Oberseite auf. Die Kappe weist einen umlaufenden äussersten Rand auf, wobei das mindestens eine Formschlusselement an einer Innenseite dieses äussersten Randes angeordnet ist. Das Formschlusselement ist somit am inneren unteren Ende der Kappe ausgebildet. Diese Form ist besonders geeignet, auf einer ebenfalls domähnlichen Pumpmembran aufgesetzt zu werden, insbesondere auf der Pumpmembran der bekannten Symphony-Pumpe.

Vorzugsweise geht der domähnliche Grundkörper in einen sich nach aussen erweiternden abgeschrägten Randbereich über, welcher in einen zylinderförmigen Randbereich übergeht, wobei der zylinderförmige Randbereich im äussersten Rand endet. Der umlaufende Anlagebereich ist vorzugsweise im Übergangsbereich vom Grundkörper in den abgeschrägten Randbereich ausgebildet. Auch diese Ausbildung ermöglicht eine optimale Verwendung mit einer Pumpmembran, welche selber einen zylinderförmigen Kragen oder Mantel aufweist, wie dies beispielsweise bei der Pumpmembran der Symphony-Pumpe der Fall ist.

Vorzugsweise ist der äusserste Rand kreisförmig ausgebildet.

Das mindestens eine Formschlusselement ist als Schnappelement ausgebildet und es ist vorzugsweise ein nach innen gerichteter Wulst. Vorzugsweise ist das mindestens eine Formschlusselement die Innenseite der Kappe umlaufend ausgebildet. Vorzugsweise sind mehrere Formschlusselemente vorhanden, welche durch Unterbrechungen voneinander getrennt sind. Vorzugsweise sind drei Formschlusselemente und drei Unterbrechungen vorhanden. Vorzugsweise sind die drei Formschlusselemente identisch ausgebildet. Dieser segmentartige Formschluss kann in geringem Masse bei erhöhtem Überdruck ein Entweichen von Luft aus der Kammer ermöglichen.

In einer bevorzugten Ausführungsform sind somit drei Schnappsegmente vorhanden, welche gleichmässig verteilt über dem inneren Umfang der Kappe und auf gleicher Höhe verlaufen. Die Formschlusselemente dienen zur korrekten Positionierung der Kappe auf der Pumpmembran. Üblicherweise wirken sie unabhängig von der Drehposition der Kappe relativ zur Pumpmembran. Für eine Orientierung bezüglich einer Drehposition können auch andere Elemente, wie beispielsweise ein Sauganschluss, vorhanden sein, welche jedoch keinen Einfluss auf die Dichtheit der Verbindung zwischen Kappe und Pumpmembran haben.

In einer weiteren Ausführungsform weist die Brustpumpe zum Abpumpen menschlicher Muttermilch eine motorisch angetriebene flexible Pumpmembran und eine auf diese stülpbare steife Kappe auf, wodurch zwischen der Pumpmembran und der Kappe eine Pumpkammer zur Erzeugung eines Unterdrucks gebildet ist. Die Kappe ist über eine Saugleitung mit einer Brusthaubeneinheit verbindbar oder verbunden. Der in der Pumpkammer erzeugte Unterdruck ist in die Brusthaubeneinheit übertragbar. Die Kappe ist durch mittelbares oder
unmittelbares Aufliegen auf der Pumpmembran dicht mit dieser verbunden, um die Pumpkammer zu bilden. In der Kappe ist ein Entlüftungsventil vorhanden, welches die Pumpkammer mit einer Aussenseite verbindet.

Diese Art der Entlüftung ist vorteilhaft, da das Ventil nicht in der Pumpmembran angeordnet sein muss. Dadurch kann sich die Pumpmembran besser bewegen und verformen. Die Saugkurven lassen sich einfacher und optimierter erstellen.

Dieses Entlüftungsventil kann mit der in diesem Text beschriebenen Kappe verwendet werden oder auch mit einer anderen Kappe. Beispielsweise kann es mit einer Kappe verwendet werden, welche nur unter Verwendung einer Schutzmembran oder einem anderen zwischen Kappe und Pumpmembran angeordneten Teil dicht und/oder fest auf der Pumpmembran aufliegt, dadurch die Pumpkammer bildet und dadurch ohne weitere Verbindungsstellen mit dem Gehäuse der Pumpe verbunden ist. Insbesondere kann das Entlüftungsventil mit einer bekannten Kappe der Symphony-Pumpe verwendet werden, indem die Kappe entsprechend mit dem Ventil ergänzt wird. Auch bei dieser Kappe sind keine weiteren Befestigungsmittel notwendig. Insbesondere muss die Kappe nicht mit Fixierungsmitteln wie Schrauben und Einrasthaken am Gehäuse der Pumpe fixiert werden. Die Kappe ist lediglich mit der Pumpmembran verbunden.

Weitere Ausführungsformen sind in den abhängigen Ansprüchen angegeben.

### KURZE BESCHREIBUNG DER ZEICHNUNGEN

Bevorzugte Ausführungsformen der Erfindung sind im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung einer erfindungsgemässen Brustpumpe mit zwei erfindungsgemässen Kappen;
- Figur 2: die Brustpumpe gemäss Figur 1 mit den zwei Kappen im angehobenen Zustand;
- Figur 3: eine perspektivische Darstellung der Kappe gemäss Figur 1 mit Saugschlauch und einer Pumpmembran mit Antriebsachse;
- Figur 4: eine Explosionsdarstellung der erfindungsgemässen Kappe und der Pumpmembran in einer Ansicht von oben;
- Figur 5: eine Explosionsdarstellung der erfindungsgemässen Kappe und der Pumpmembran in einer Ansicht von unten;
- Figur 6: einen Querschnitt durch die Kappe und die Pumpmembran im zusammen gesetzten Zustand;
- Figur 7: einen vergrösserten Ausschnitt gemäss Figur 6;
- Figur 8: einen Querschnitt durch die Kappe gemäss Figur 1;
- Figur 9: einen anderen Querschnitt durch die Kappe gemäss Figur 1;
- Figur 10: eine Ansicht der Kappe gemäss Figur 1 von unten;
- Figur 11: einen vergrösserten Ausschnitt durch die Kappe gemäss Figur 1 und durch ein kappenseitiges Kopplungsteil in Explosionsdarstellung;
- Figur 12: den Ausschnitt der Kappe gemäss Figur 11 mit dem Kopplungsteil im zusammen gesetzten Zustand;
- Figur 13: eine perspektivische Darstellung einer erfindungsgemässen Kappe in einer zweiten Ausführungsform;
- Figur 14: einen Querschnitt durch die Kappe gemäss Figur 13 und
- Figur 15: einen vergrösserten Ausschnitt eines Querschnitts durch eine erfindungsgemässe Kappe in einer dritten Ausführungsform.

Gleiche Teile sind mit gleichen Bezugszeichen versehen.

### BESCHREIBUNG BEVORZUGTER AUSFÜHRUNGSFORMEN

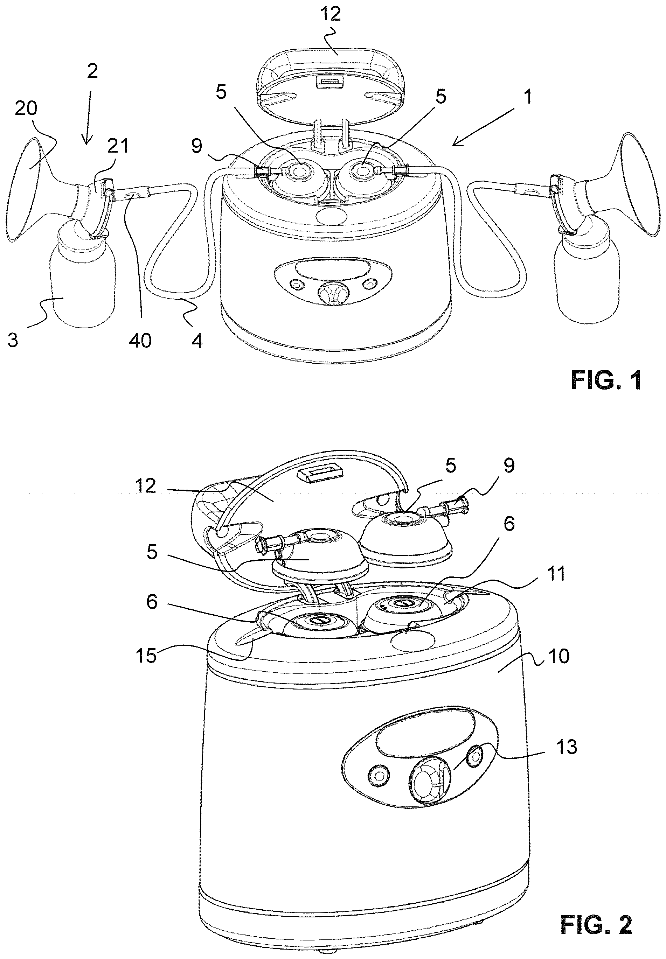

In Figur 1 ist eine Brustpumpeneinheit mit einer erfindungsgemässen Brustpumpe 1 dargestellt. Die Brustpumpe 1 weist ein Gehäuse 10 auf, welches auf seiner oberen Seite eine Vertiefung 11 besitzt. In dieser Vertiefung 11 sind zwei flexible Pumpmembranen 6 angeordnet, welche über eine Antriebsachse 14 mit einem mittels eines Elektromotors angetriebenen Pumpmechanismus verbunden sind.

Jede Pumpmembran 6 weist eine um ihren Umfang verlaufende Fixierungsplatte 62 und auf ihrer Unterseite Einrasthaken 64 auf. Die Antriebsachse 14 ist mit der Pumpmembran 6 ebenfalls fest, aber lösbar verbunden, wobei hierfür eine entsprechende Befestigungsnut 66 in der Pumpmembran vorhanden ist. Vorzugsweise befindet sich die Befestigungsnut 66 im oberen mittigen Bereich des Grundkörpers 60, so dass die Antriebsachse 14 entlang der Längsmittelachse der Pumpmembran 6 wirkt. Dank dieser Verbindungen ist die Pumpmembran im Gehäuse 10 der Brustpumpe 1 fixiert gehalten und wird üblicherweise vom Benützer nicht aus dem Gehäuse 10 entfernt.

Der Pumpmechanismus ist im Gehäuse 10 angeordnet. Die übrigen erwähnten Elemente sind in den Figuren 2, 3 und 6 dargestellt.

Ein Deckel 12 der Brustpumpe 1 verschliesst die Vertiefung 11 bzw. macht sie von aussen zugänglich. Ein auf einer äusseren Fläche des Gehäuses angeordnetes Anzeige- und Bedienfeld 13 ermöglicht dem Benützer, die Pumpe zu aktivieren und ihre gewünschte Funktionsweise zu wählen.

Die Brustpumpe umfasst ferner mindestens eine steife Kappe 5. Hier sind zwei steife Kappen 5 vorhanden. Die Kappen 5 sind vorzugsweise einstückig aus Kunststoff gefertigt.

Je eine Kappe 5 ist lösbar über eine der zwei Pumpmembranen 6 gestülpt.

Jede Kappe 5 ist mit einem Saugschlauch 4 entweder lösbar oder fest und nicht zerstörungsfrei lösbar verbunden. Der Saugschlauch 4 führt zu einer Brusthaubeneinheit 2, wobei er vorzugsweise mittels eines brusthaubenseitigen Kopplungsteils 40 in diese einsteckbar ist.

Die Brusthaubeneinheit 2 weist einen Konnektor 21 zur Verbindung mit dem Saugschlauch 4 und eine Brusthaube 20 zum Sammeln der abzupumpenden Mutterbrust auf. Die Brusthaube 20 ist entweder einstückig mit dem Konnektor 21 verbunden oder lösbar in diesem gehalten. Der Konnektor 21 weist ferner einen Milchanschluss zur Verbindung mit einem Milchsammelbehälter, hier einer Milchflasche 3, auf.

Die Verbindung zwischen Vakuum- oder Saugschlauch 4 und Kappe 5 erfolgt über ein kappenseitiges Kopplungsteil 9. Dieses Kopplungsteil 9 und/oder der Saugschlauch 4 lässt sich in eine Nut 15 des Gehäuses 10 legen, damit der Deckel 12 bei eingelegter Kappe 5 geschlossen werden kann. Die Vorrichtung funktioniert jedoch auch bei geöffnetem Deckel 12. Die Kappen 5 sind nicht mit dem Deckel 12 verbunden, sondern sie sind vom Deckel 12 und vom Gehäuse 10 unabhängige und getrennte Teile.

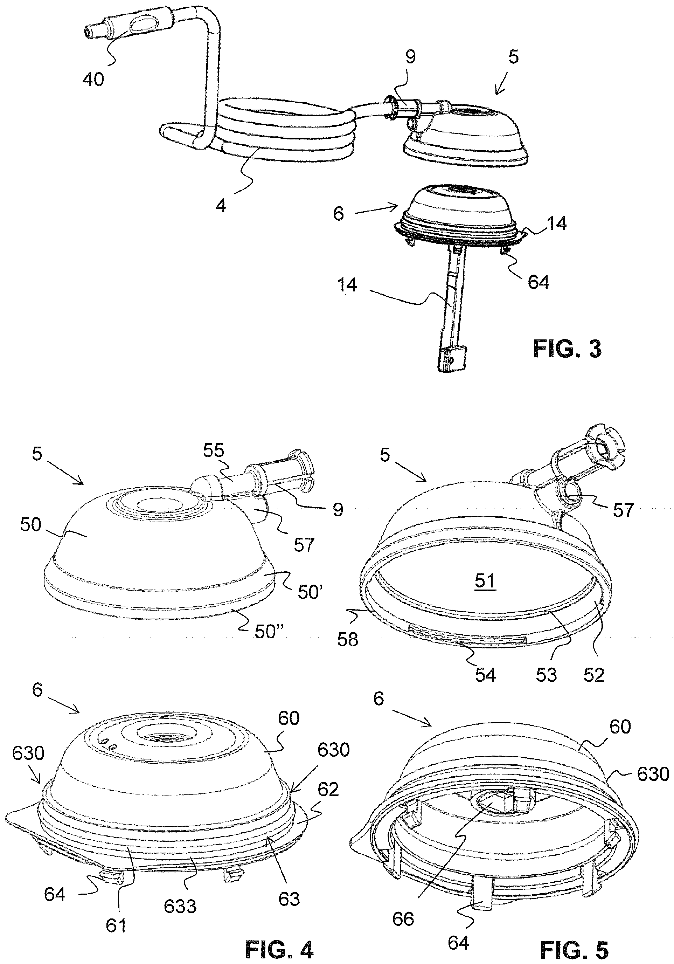

In den Figuren 4 und 5 sind Kappe 5 und Pumpmembran 6 einzeln dargestellt. Die Kappe 5 weist einen domförmigen Grundkörper 50 mit rundem Querschnitt auf. Der Grundkörper 50 geht an seinem unteren offenen Ende in einen abgeschrägten und sich nach aussen erweiternden Bereich 50' über. Dieser abgeschrägte Bereich 50' endet in einem kreiszylinderförmigen Bereich 50", welcher das untere Ende der Kappe 5 bildet und somit in einem äussersten Rand endet.

An der Kappe 5 ist eine Schlauchaufnahme 55 angeordnet, welche hier zugleich als Griff ausgebildet ist, damit sich die Kappe 5 einfach in die Vertiefung des Gehäuses 1 einsetzen und somit auf die Pumpmembran 6 aufsetzen und ebenso einfach wieder daraus bzw. davon entfernen lässt. Die Schlauchaufnahme 55 ist mit dem kappenseitigen Kopplungsteil 9 verbunden.

An der unteren Seite der Schlauchaufnahme 55 ist eine Ventilaufnahme 57 vorhanden. Diese kann jedoch auch in einem anderen Bereich der Kappe 5 angeordnet sein.

Die Kappe 5 ist innen hohl ausgebildet. Der Hohlraum ist in Figur 5 mit der Bezugsziffer 51 bezeichnet. Die Kappe 5 und somit der Hohlraum 51 sind nach unten offen ausgebildet, wobei die Zugangsöffnung zum Hohlraum 51 dem inneren Durchmesser des äussersten Randes entspricht.

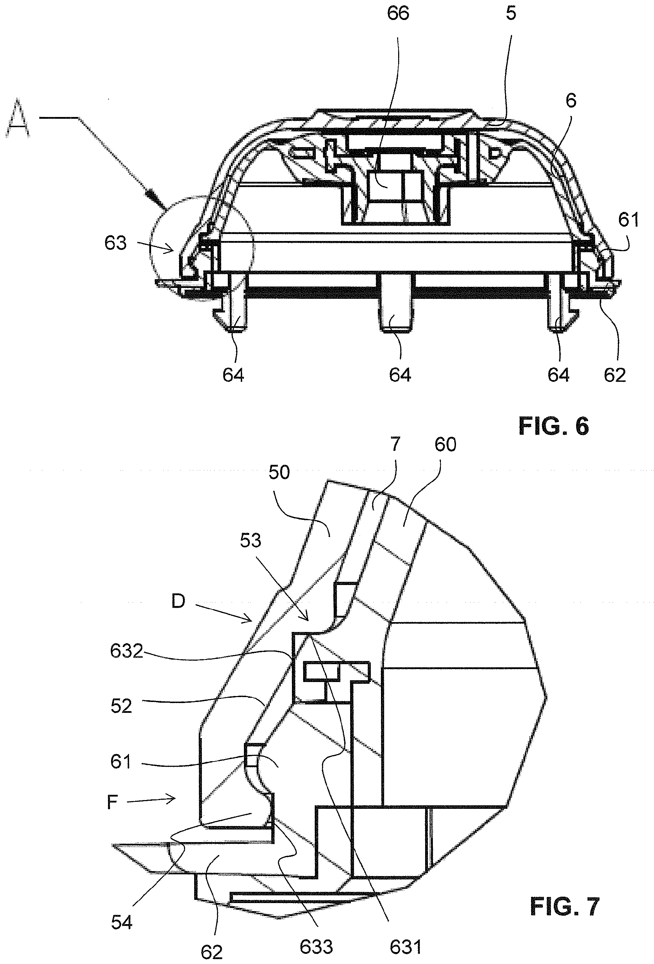

Ein in sich geschlossener Anlagebereich 53, hier durch eine innere Wulst als Schulter ausgebildet, umläuft eine innere Oberfläche der Kappe 5. Diese Schulter 53 befindet sich vorzugsweise im Bereich des Übergangs vom Grundkörper 50 zum abgeschrägten Bereich 50' und ist in den Hohlraum 51 hinein gerichtet. Wie in Figur 7 gut erkennbar ist, weist die Schulter 53 auf ihrer Unterseite eine annähernd horizontal verlaufende, umlaufende Fläche auf. Diese dient als Anschlag, wenn sie auf einer Stufe 630, genauer auf einer horizontalen Stufenfläche 631 oder Sims, der Pumpmembran 6 aufliegt. Der Benützer merkt dadurch, dass die Kappe 5 korrekt auf der Pumpmembran 6 aufgesetzt ist.

Auf der Innenseite des kreisförmigen hohlzylindrischen Bereichs 50" ist mindestens ein Formschlusselement 54 vorhanden. Dieses Formschlusselement ist als Schnappelement 54 ausgebildet. Es weist vorzugsweise die Form einer nach innen gerichteten Wulst auf, welche nahe oder angrenzend an den äussersten Rand, d.h. die nach unten gerichtete Stirnfläche der Kappe 5, angeordnet ist. Sind mehrere Schnappelemente 54 vorhanden, so verlaufen sie vorzugsweise im gleichen und/oder gleichbleibenden Abstand zum äussersten Rand.

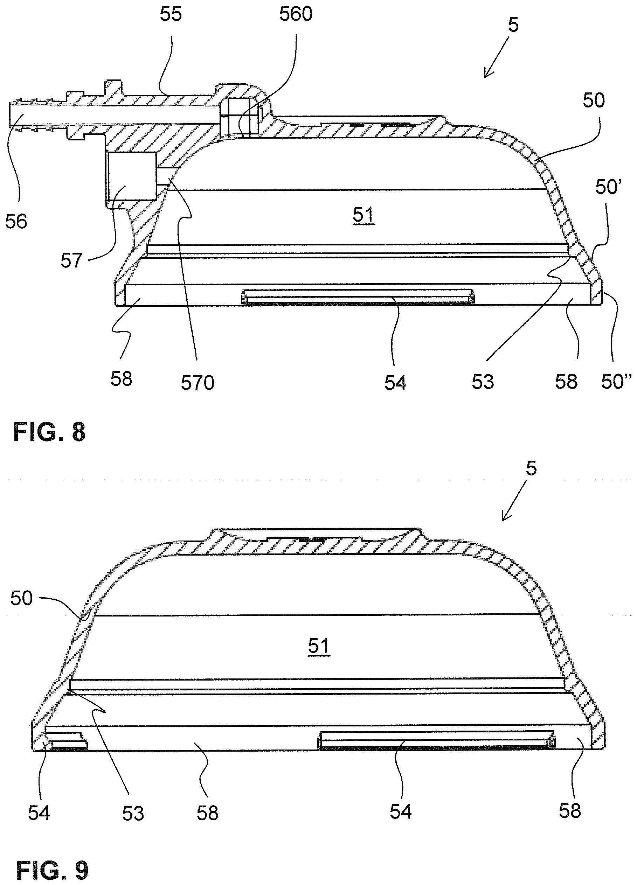

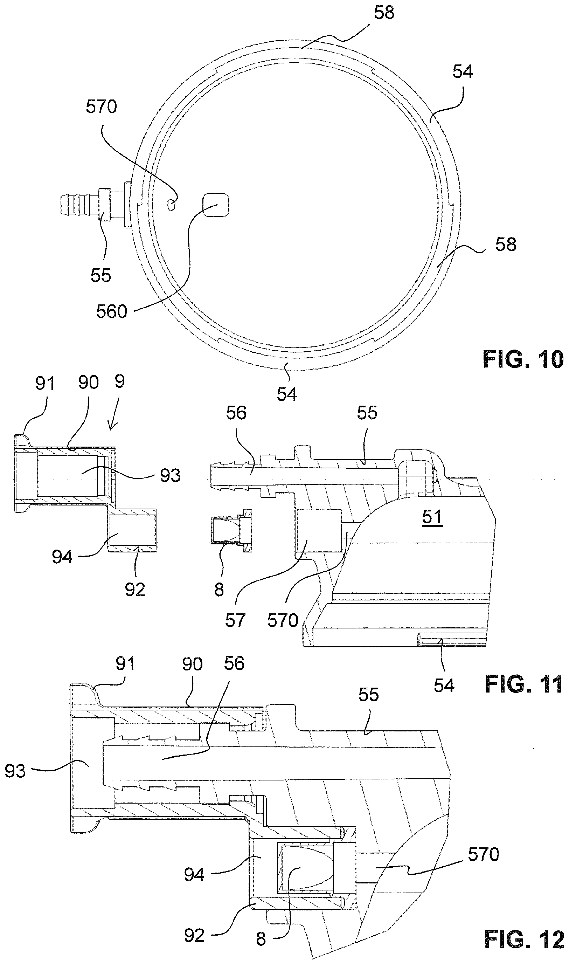

Das mindestens eine Schnappelement 54 umläuft den inneren Mantel der Kappe 5. Vorzugsweise sind mehrere Schnappelemente 54 vorhanden, welche durch Unterbrechungen 58 voneinander getrennt sind. Dies ist in den Figuren 9 und 10 gut erkennbar.

Zwischen der Schulter 53 und den Schnappelementen 54 ist ein Kanal 52 ausgebildet, wie dies in den Figuren 5 und 7 gut erkennbar ist.

In Figur 8 ist die Ventilaufnahme 57 dargestellt. Sie ist durch eine nach aussen führende kreiszylinderförmige Öffnung gebildet, welche in einen Luftkanal 570 mit kleinerem Durchmesser übergeht. Der Luftkanal 570 mündet in den Hohlraum 51 der Kappe 5. Oberhalb der Ventilaufnahme 57 ist die Schlauchaufnahme 55 an der Kappe 5 angeformt, Sie weist einen Stutzen 56 zur Verbindung mit dem kappenseitigen Kopplungsteil 9 auf. Der Stutzen 56 weist einen Kanal auf, welcher mit einem Ende nach aussen führt und mit einem anderen Ende in eine in den Hohlraum 51 der Kappe 5 mündende Saugöffnung 560 übergeht.

Das kappenseitige Kopplungsteil 9 ist in den Figuren 11 und 12 gut erkennbar. Es weist einen Saugstutzen 90 mit einem Saugkanal 93 zur Verbindung mit dem Stutzen 56 und der Saugöffnung 560 der Kappe 5 auf.

Vorzugsweise wird der Saugstutzen 90 über den Stutzen 56 geschoben, wobei vorzugsweise Stufen und Rückhaltestrukturen der Schlauchaufnahme 55 und des Kopplungsteils 9 verhindern, dass das Kopplungsteil 9 zerstörungsfrei wieder entfernt werden kann. Ein Überstreifschutz 91, hier in Form einer kreisförmigen Struktur mit Unterbrechungen in ihrem Umfang, verhindern, dass ein Saugschlauch mit grösserem Durchmesser über dieses Kopplungsteil geschoben werden kann.

Am kappenseitigen Kopplungsteil 9 ist vorzugsweise einstückig ein Ventilstutzen 92 mit einem Luftkanal 94 angeformt. Er befindet sich vorzugsweise unterhalb des Saugstutzens 90, wobei der Luftkanal 94 parallel unterhalb des Saugkanals 93, jedoch getrennt von diesem, verläuft.

Dieser Ventilstutzen 92 dient der Aufnahme eines Entlüftungsventils 8, hier eines Rückschlagventils, vorzugsweise eines Entenschnabelventils. Der Ventilstutzen 92 lässt sich mit dem Rückschlagventil 8 in die Ventilaufnahme 57 schieben, wenn das kappenseitige Kopplungsteil 9 auf der Schlauchaufnahme 55 befestigt wird. Dadurch ist das Rückschlagventil 8 in der Kappe 5 fixiert gehalten. Es öffnet vom Hohlraum 51 nach aussen, also bei Überdruck im Hohlraum 51 gegenüber der Atmosphäre ausserhalb der Kappe 5. Die Kappe 5 ist ansonsten vorzugsweise einstückig ausgebildet.

In den Figuren 4 und 5 ist ferner die Pumpmembran 6 gut erkennbar. Sie weist einen domförmigen Grundkörper 60 auf, welcher vorzugsweise das Gegenstück zum domförmigen Hohlraum 51 der Kappe 5 bildet und somit an der Innenwandung der Kappe 5 anliegt. Der Grundkörper 60 geht in seinem unteren Bereich in einer Stufe in den umlaufenden und in sich geschlossenen Kragen 63 über. Der Kragen 63 weist die Stufe 630 und somit die vorzugsweise horizontal verlaufende, nach oben gerichtete Stufenfläche 631 bzw. den Sims auf. Der Kragen 63 ist in seinem oberen Bereich vorzugsweise zylinderförmig ausgebildet und liegt in Form einer vertikal verlaufenden Stufenfläche 632 vor. Anschliessend weist er eine äussere Wulst 61 auf, welche nach aussen vorsteht und umlaufend sowie in sich geschlossen ausgebildet ist. Vorzugsweise weist der Kragen 63 einen der Fixierungsplatte 62 benachbarten unteren Kragenbereich 633 auf, welcher unterhalb der Wulst 61 liegt und einen grösseren äusseren Durchmesser besitzt als der Bereich der vertikalen Stufenfläche 632.

In den Figuren 6 und 7 sind die Kappe 5 und die Pumpmembran 6 im zusammengebauten Zustand dargestellt. Die Kappe 5 ist über die Pumpmembran 6 gestülpt, wobei die Schnappelemente 54 unter die äussere Wulst 61 ragen und diese somit hintergreifen. Dadurch ist die Kappe 5 auf die Pumpmembran 6 formschlüssig eingeschnappt. Der formschlüssige Fixierungsbereich ist in Figur 7 mit dem Bezugszeichen F gekennzeichnet.

Ist die Kappe 5 montiert und stehen die Schnappelemente 54 mit der äusseren Wulst 61 im Eingriff, so liegt die Schulter 53 auf der oberen Stufenfläche 631 des Kragens 63 auf. Wie in Figur 7 erkennbar ist, bildet dieser Bereich zusammen mit dem benachbarten Bereich des Kanals 52 einen Reibschluss und somit den Dichtbereich D.

Die Kappe 5 ist somit ohne weitere zusätzliche Teile direkt auf der Pumpmembran 6 dichtend gehalten. Zwischen Pumpmembran 6 und Kappe 5 ist im Hohlraum 51 der Kappe 5 eine Pumpkammer 7 gebildet. Das Volumen der Pumpkammer 7 ist minimal oder Null, wenn sich die Pumpmembran 6 in ihrer domförmigen Grundform befindet.

Diese Grundform wird durch den Pumpmechanismus verändert, indem die Antriebsachse 14 den mittleren Bereich des Grundkörpers 60 der Pumpmembran 6 nach unten zieht. Der Grundkörper 60 wird dadurch verformt. Stösst die Antriebsachse 14 den Grundkörper 60 wieder nach oben, so nimmt er wieder seine ursprüngliche Form an. Der Kragen 63 wird vorzugsweise nicht verformt sondern ist steif ausgebildet. Durch die Formänderung des Grundkörpers 60 ändert sich das Volumen der Pumpkammer 7, ein Unterdruck wird zyklisch erzeugt und die Druckveränderung wird in die Brusthaubeneinheit 2 und an die Brusthaube 20 übertragen.

Falls sich beim ersten Hub die Pumpmembran 6 noch nicht in ihrer Grundform befindet, sondern die Luft in der Pumpkammer 7 zuerst komprimiert wird, kommt das Entlüftungsventil 8 zum Einsatz. Die Luft kann aus dem Ventil 8 entweichen und die Pumpe ist unmittelbar darauf einsatzfähig, ohne dass der Benutzer etwas gemerkt hat. Ist beim ersten Hub der Druck in der Druckkammer 7 zu hoch, so entweicht die Luft zudem durch die Unterbrechungen zwischen den Schnappelementen 54.

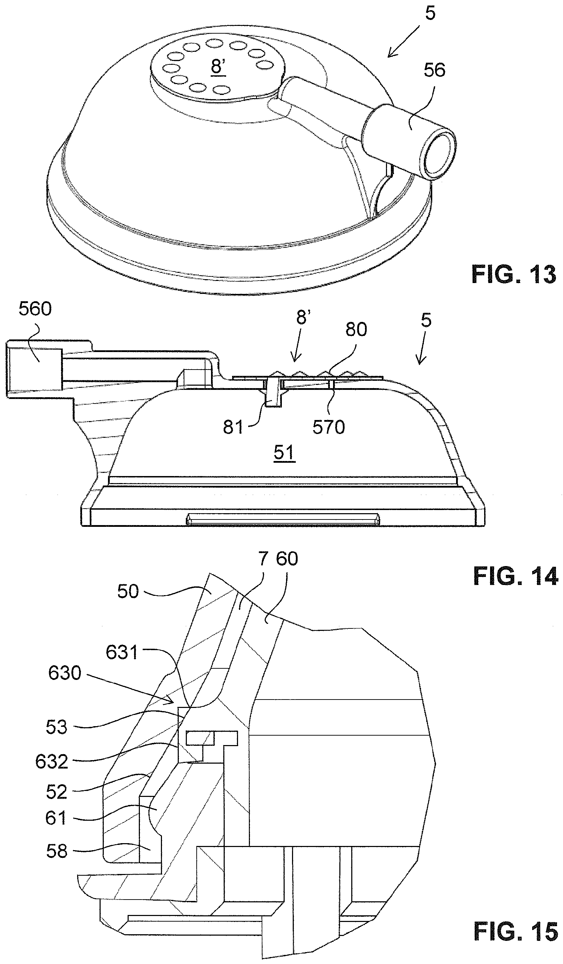

In den Figuren 13 und 14 ist eine Kappe 5 mit einem alternativen Entlüftungsventil 8' dargestellt. Es ist eine Membranklappe 80 mit einem Befestigungsstumpf 81, welcher in einer Ausnehmung in der oberen Wand der Kappe 5 gehalten ist. Die Membranklappe 80 überdeckt dabei den Luftkanal 570 der Kappe 5, welcher die obere Wand der Kappe 5 durchsetzt und vom Hohlraum 51 nach aussen führt.

In Figur 15 ist eine weitere Ausführungsform dargestellt, welche ohne Entlüftungsventil ausgebildet ist oder welche mit einem der Ventile 8, 8' gemäss den oben beschriebenen Ausführungsformen versehen sein kann. In dieser Ausführungsform weist die Kappe 5 auf ihrer Innenseite keine Schulter auf, sondern eine im Querschnitt schräg und stetig verlaufende Anlagefläche, welche den Anlagebereich 53 bildet. Diese umlaufende Anlagefläche 53 bildet mit der Kante der Stufe 630 einen Reibschluss. Am unteren Rand der Kappe 5 sind wiederum die bereits beschriebenen Schnappelemente 54 vorhanden, wobei in der Darstellung gemäss Figur 15 der Schnitt durch eine der Unterbrechungen 58 dargestellt ist.

Die erfindungsgemässe Brustpumpe und die erfindungsgemässe steife Kappe ermöglichen eine luftdichte und einfach erstellbare Verbindung zwischen der Kappe und einer flexiblen Pumpmembran, um eine Pumpkammer auszubilden.

**BEZUGSZEICHENLISTE**

| | | | |
|---|---|---|---|
| 1 | Brustpumpe | | |
| 10 | Gehäuse | 6 | Pumpmembran |
| 11 | Vertiefung | 60 | Grundkörper |
| 12 | Deckel | 61 | äussere Wulst |
| 13 | Bedien- und Anzeigefeld | 62 | Fixierungsplatte |
| 14 | Antriebsachse | 63 | Kragen |
| | | 630 | Stufe |
| 2 | Brusthaube | 631 | horizontale Stufenfläche/Sims |
| 20 | Trichter | 632 | vertikal verlaufende Stufenfläche |
| 21 | Konnektor | 633 | unterer Kragenbereich |
| | | 64 | Einrasthaken |
| 3 | Milchsammelbehälter | 66 | Befestigungsnut |
| 4 | Saugschlauch | | |
| 40 | brusthaubenseitiges Kopplungsteil | 7 | Pumpkammer |
| 5 | Kappe | 8 | Entlüftungsventil |
| 50 | Grundkörper | 8' | Entlüftungsventil |
| 50' | abgeschrägter Randbereich | 80 | Membranklappe |
| 50" | zylinderförmiger Randbereich | 81 | Befestigungsstumpf |
| 51 | Hohlraum | | |
| 52 | Kanal | 9 | kappenseitiges Kopplungsteil |
| 53 | Anlagebereich | 90 | Saugstutzen |
| 54 | Schnappelement | 91 | Überstreifschutz |
| 55 | Griff | 92 | Ventilstutzen |
| 56 | Stutzen | 93 | Saugkanal |
| 560 | Saugöffnung | 94 | Luftkanal |
| 57 | Ventilaufnahme | | |
| 570 | Luftkanal | D | Dichtbereich |
| 58 | Unterbrechung | F | Fixierungsbereich |

## Patentansprüche

1. Brustpumpe zum Abpumpen menschlicher Muttermilch mit einer motorisch angetriebenen flexiblen Pumpmembran (6) und einer auf diese stülpbare steifen Kappe (5), wodurch zwischen Pumpmembran (6) und Kappe (5) eine Pumpkammer (7) zur Erzeugung eines Unterdrucks gebildet ist, wobei die Kappe (5) über eine Saugleitung (4) mit einer Brusthaubeneinheit (2) verbindbar oder verbunden ist und wobei der in der Pumpkammer (7) erzeugte Unterdruck in die Brusthaubeneinheit (2) übertragbar ist, wobei die Pumpmembran (6) einen umlaufenden Kragen (63) aufweist mit einer Stufe (630) und mit einem seitlich vorstehenden Vorsprung (61) und wobei die Kappe (5) auf ihrer Innenseite einen umlaufenden Anlagebereich (53) aufweist,
wobei die Kappe (5) auf ihrer Innenseite mindestens ein Formschlusselement (54) aufweist, welches beabstandet zum umlaufenden Anlagebereich (53) angeordnet ist und welches mit dem Vorsprung (61) der Pumpmembran einen Formschluss (F) bildet, wodurch die Kappe (5) eingeschnappt auf der Pumpmembran (6) aufliegt, und wobei der Anlagebereich (53) der Kappe (5) und die Pumpmembran (6) im Bereich der Stufe (630) in einem gemeinsamen Reibschluss stehen und eine Dichtung (D) bilden, so dass das eingeschnappte Aufliegen der Kappe (5) auf der Pumpmembran (6) ausreicht, um die Kappe (5) dichtend auf der Pumpmembran (6) zu halten, **dadurch gekennzeichnet, dass** das mindestens eine Formschlusselement ein Schnappelement (54) ist, welches unter den Vorsprung (61) der Pumpmembran (6) eingreift.

2. Brustpumpe nach Anspruch 1, wobei zwischen dem Anlagebereich (53) und dem mindestens einen Formschlusselement (54) ein umlaufender Kanal (52) gebildet ist, welcher den Kragen (63) der Pumpmembran (6) umgibt und welcher den Vorsprung (61) aufnimmt.

3. Brustpumpe nach einem der Ansprüche 1 oder 2, wobei zwischen der Kappe (5) und der Pumpmembran (6) keine zusätzlichen Teile angeordnet sind.

4. Brustpumpe nach einem der Ansprüche 1 bis 3, wobei ein Entlüftungsventil (8, 8') vorhanden ist, welches die Pumpkammer (7) mit einer Aussenseite verbindet.

5. Brustpumpe nach Anspruch 4, wobei das Entlüftungsventil (8, 8') in der Kappe (5) angeordnet ist.

6. Brustpumpe nach einem der Ansprüche 1 bis 5, wobei ein Entlüftungsventil (8, 8') vorhanden ist, welche in einem die Saugleitung (4) mit der Kappe (5) verbindenden Stecker (9) oder in der Brusthaubeneinheit (2) oder in der Saugleitung (4) angeordnet ist.

7. Steife Kappe zur Verwendung in einer Brustpumpe gemäss einem der Ansprüche 1 bis 6, wobei die Kappe (5) über eine Saugleitung (4) mit einer Brusthaubeneinheit (2) verbindbar oder verbunden ist und wobei die Kappe (4) auf ihrer Innenseite einen umlaufenden Anlagebereich (53) aufweist,
wobei die Kappe (5) auf ihrer Innenseite mindestens ein Formschlusselement (54) aufweist, welches beabstandet zum umlaufenden Anlagebereich (53) angeordnet ist zur Bildung des Formschlusses (F) mit dem Vorsprung (61) der Pumpmembran (6) der Brustpumpe, wodurch die Kappe eingeschnappt auf der Pumpmembran aufliegen kann, und wobei die Kappe (5) im Bereich des Anlagebereichs (53) zur Bildung des Reibschlusses (D) mit der Pumpmembran (6) ausgebildet ist, wobei der Reibschluss (D) im Bereich der Stufe (630) der Pumpmembran (6) erfolgt, so dass ein eingeschnapptes
Aufliegen der Kappe (5) auf der Pumpmembran (6) ausreicht, um die Kappe (5) dichtend auf der Pumpmembran (6) zu halten, **dadurch gekennzeichnet, dass** das mindestens eine Formschlusselement (54) als Schnappelement ausgebildet ist und durch einen nach innen gerichteten Wulst gebildet ist.

8. Kappe nach Anspruch 7, wobei sie einen domähnlichen Grundkörper (50) und einen umlaufenden äussersten Rand aufweist, wobei das mindestens eine Formschlusselement (54) an einer Innenseite des äussersten Randes angeordnet ist.

9. Kappe nach Anspruch 8, wobei der domähnliche Grundkörper (50) in einen sich nach aussen erweiternden abgeschrägten Randbereich (50') übergeht, welche in einen zylinderförmigen Randbereich (50") übergeht, wobei der zylinderförmige Randbereich (50") im äussersten Rand endet.

10. Kappe nach Anspruch 9, wobei der umlaufende Anlagebereich (53) im Übergangsbereich vom Grundkörper (50) in den abgeschrägten Randbereich (50') ausgebildet ist.

11. Kappe nach einem der Ansprüche 8 bis 10, wobei der äusserste Rand kreisförmig ausgebildet ist.

12. Kappe nach einem der Ansprüche 7 bis 11, wobei das mindestens eine Formschlusselement (54) einen Umfang einer Innenseite der Kappe (5) umlaufend ausgebildet ist, wobei im Falle von mehreren Formschlusselementen (54) diese im Umfang der Innenseite durch Unterbrechungen (58), vorzugsweise von genau drei Unterbrechungen (58), voneinander getrennt sind.

## Claims

1. Breast pump for expressing human breast milk having a motor-driven flexible pump diaphragm (6) and a rigid cap (5) that can be put thereover, whereby a pump chamber (7) for generating a negative pressure is formed between pump diaphragm (6) and cap (5), the cap (5) being connectable or connected via a suction line (4) to a breast shield unit (2), and the negative pressure generated in the pump chamber (7) being transferrable into the breast shield unit (2), the pump diaphragm (6) having a circumferential collar (63) with a step (630) and having a laterally projecting protrusion (61), and the cap (5) having, on the inner side thereof, a circumferential bearing region (53),
wherein the cap (5) has, on the inner side thereof, at least one form-fit element (54), which is arranged at a distance from the circumferential bearing region (53) and which together with the protrusion (61) of the pump diaphragm forms a form fit (F), whereby the cap (5) rests on the pump diaphragm (6) in a manner snapped into place, and wherein the bearing region (53) of the cap (5) and the pump diaphragm (6) in the region of the step (630) are involved in a joint frictional connection and form a seal (D), such that the resting of the cap (5) on the pump diaphragm (6) in a manner snapped into place is sufficient to hold the cap (5) in a sealing manner on the pump diaphragm (6), **characterized in that** the at least one form-fit element is a snap-fit element (54), which engages below the protrusion (61) of the pump diaphragm (6).

2. Breast pump according to Claim 1, a circumferential channel (52) being formed between the bearing region (53) and the at least one form-fit element (54) and surrounding the collar (63) of the pump diaphragm (6) and receiving the protrusion (61).

3. Breast pump according to one of Claims 1 or 2, no additional parts being arranged between the cap (5) and the pump diaphragm (6).

4. Breast pump according to one of Claims 1 to 3, a venting valve (8, 8') being provided, which connects the pump chamber (7) to an outside.

5. Breast pump according to Claim 4, the venting valve (8, 8') being arranged in the cap (5).

6. Breast pump according to one of Claims 1 to 5, a venting valve (8, 8') being provided, which is arranged in a plug (9) connecting the suction line (4) to the cap (5) or in the breast shield unit (2) or in the suction line (4).

7. Rigid cap for use in a breast pump according to one of Claims 1 to 6, the cap (5) being connectable or connected via a suction line (4) to a breast shield unit (2), and the cap (4) having, on the inner side thereof, a circumferential bearing region (53),
wherein the cap (5), on the inner side thereof, has at least one form-fit element (54), which is arranged at a distance from the circumferential bearing region (53) in order to form the form fit (F) with the protrusion (61) of the pump diaphragm (6) of the breast pump, and whereby the cap (5) can rest on the pump diaphragm in a manner snapped into place wherein the cap (5) is designed in the region of the bearing region (53) to form the frictional connection (D) with the pump diaphragm (6), wherein the frictional connection (D) takes place in the region of the step (630) of the pump diaphragm (6), such that a resting of the cap (5) on the pump diaphragm (6) in a manner snapped into place is sufficient to hold the cap (5) in a sealing manner on the pump diaphragm (6), **characterized in that** the at least one form-fit element (54) is designed as a snap-fit element and being formed by an inwardly directed bead.

8. Cap according to Claim 7, wherein it has a dome-like main body (50) and a circumferential outermost edge, the at least one form-fit element (54) being arranged on an inner side of the outermost edge.

9. Cap according to Claim 8, the dome-like main body (50) transitioning into an edge region (50') that is chamfered in a manner widening outwardly and which transitions into a cylindrical edge region (50"), the cylindrical edge region (50") ending in the outermost edge.

10. Cap according to Claim 9, the circumferential bearing region (53) being formed in the transition region from the main body (50) into the chamfered edge region (50').

11. Cap according to one of Clams 8 to 10, the outermost edge being circular.

12. Cap according to one of Claims 7 to 11, the at least one form-fit element (54) being formed in a manner running around a periphery of an inner side of the cap (5), and, in the case of a number of form-fit elements (54), these being separated from one another in the periphery of the inner side by interruptions (58), preferably by exactly three interruptions (58).

## Revendications

1. Tire-lait, destiné à pomper le lait maternel humain, comprenant une membrane de pompage flexible (6) entraînée par moteur et un capuchon rigide (5) pouvant être retourné et pouvant être placé sur ladite membrane de pompage de manière à former une chambre de pompage (7) entre la membrane de pompage (6) et le capuchon (5) afin de générer une pression négative, le capuchon (5) étant relié ou pouvant être relié à une unité formant téterelle (2) par le biais d'un conduit d'aspiration (4), et la pression négative générée dans la chambre de pompage (7) pouvant être transférée dans l'unité formant téterelle (2), la membrane de pompage (6) comportant un collet périphérique (63) muni d'un gradin (630) et d'une saillie (61) se projetant latéralement et le capuchon (5) comportant sur son côté intérieur une région d'appui périphérique (53),
le capuchon (5) comportant sur son côté intérieur au moins un élément de liaison par complémentarité de formes (54) qui est disposé à distance de la région d'appui périphérique (53) et qui forme une liaison par complémentarité de formes (F) avec la saillie (61) de la membrane de pompage de manière à ce que le capuchon (5) vienne en appui avec encliquetage sur la membrane de pompage (6), et la région d'appui (53) du capuchon (5) et la membrane de pompage (6) forment dans la région du gradin (630) une liaison par friction commune et forment un joint d'étanchéité (D) de sorte que l'appui avec encliquetage du capuchon (5) sur la membrane de pompage (6) soit suffisant pour maintenir le capuchon (5) de manière étanche sur la membrane de pompage (6), **caractérisé en ce que** l'au moins un élément de liaison par complémentarité de formes est un élément d'encliquetage (54) qui s'engage sous la saillie (61) de la membrane de pompage (6).

2. Tire-lait selon la revendication 1, un conduit périphérique (52) étant formé entre la région d'appui (53) et l'au moins un élément de liaison par complémentarité de formes (54), lequel conduit entoure le collet (63) de la membrane de pompage (6) et reçoit la saillie (61).

3. Tire-lait selon l'une des revendications 1 et 2, aucune pièce supplémentaire n'étant disposée entre le capuchon (5) et la membrane de pompage (6).

4. Tire-lait selon l'une des revendications 1 à 3, une soupape de ventilation (8, 8') étant prévue qui relie la chambre de pompage (7) à un extérieur.

5. Tire-lait selon la revendication 4, la soupape de ventilation (8, 8') étant disposée dans le capuchon (5).

6. Tire-lait selon l'une des revendications 1 à 5, une soupape de ventilation (8, 8') étant prévue qui est disposée dans un connecteur (9) reliant le conduit d'aspiration (4) au capuchon (5) ou dans l'unité formant téterelle (2) ou dans la conduite d'aspiration (4).

7. Capuchon rigide destiné à être utilisé dans un tire-lait selon l'une quelconque des revendications 1 à 6, le capuchon (5) étant relié ou pouvant être relié à une unité formant téterelle (2) par le biais d'un conduit d'aspiration (4), et le capuchon (4) comportant sur son côté intérieur une région d'appui périphérique (53),
le capuchon (5) comportant sur son côté intérieur au moins un élément de liaison par complémentarité de formes (54) qui est disposé à distance de la région d'appui périphérique (53) pour former la liaison par complémentarité de formes (F) avec la saillie (61) de la membrane de pompage (6) du tire-lait de manière à ce que le capuchon puisse venir en appui avec encliquetage sur la membrane de pompage, et le capuchon (5) étant conçu dans la région d'appui (53) pour former la liaison par friction (D) avec la membrane de pompage (6), la liaison par friction (D) se faisant dans la région du gradin (630) de la membrane de pompage (6) de manière à ce qu'un appui avec encliquetage du capuchon (5) sur la membrane de pompage (6) soit suffisant pour maintenir le capuchon (5) de façon étanche sur la membrane de pompage (6), **caractérisé en ce que** l'au moins un élément de liaison par complémentarité de formes (54) est conçu comme un élément à encliquetage et est formé par un bourrelet dirigé vers l'intérieur.

8. Capuchon selon la revendication 7, le capuchon comportant un corps de base (50) en forme de dôme et un bord périphérique le plus à l'extérieur, l'au moins un élément de liaison par complémentarité de formes (54) étant disposé sur un côté intérieur du bord le plus à l'extérieur.

9. Capsule selon la revendication 8, le corps de base (50) en forme de dôme se transformant en une région de bord biseautée (50') qui s'élargit vers l'extérieur et qui se transforme en une région de bord cylindrique (50"), la région de bord cylindrique (50") se terminant dans le bord le plus à l'extérieur.

10. Capsule selon la revendication 9, la région d'appui périphérique (53) étant formée dans la région de transition depuis le corps de base (50) jusque dans la région de bord biseauté (50').

11. Capsule selon l'une des revendications 8 à 10, le bord le plus à l'extérieur étant circulaire.

12. Capsule selon l'une des revendications 7 à 11, l'au moins un élément de liaison par complémentarité de formes (54) formant circonférentiellement une périphérie d'un côté intérieur du capuchon (5), dans le cas de plusieurs éléments de liaison par complémentarité de formes (54) ceux-ci étant séparés les uns des autres sur la périphérie du côté intérieur par des interruptions (58), de préférence exactement trois interruptions (58).
